# EUROPEAN PATENT APPLICATION

(11) **EP 4 501 134 A1**
(43) Date of publication of application: **05.02.2025**
(21) Application number: 23780852.2
(22) Date of filing: 30.03.2023
(51) Int. Cl.: A23L 27/00, A23L 2/00, A23L 2/38, A23L 2/52, A23L 2/66, A23L 5/00, A23L 11/65, C12P 1/00, C12P 7/26

(54) **MASKING AGENT**

(30) Priority: 31.03.2022 JP 2022058519
(71) Applicant: Fuji Oil Holdings Inc., Izumisano-shi, Osaka 598-8540 (JP); Amano Enzyme Inc., Nagoya-shi Aichi 460-8630 (JP)
(72) Inventor: BABA, Misato, Izumisano-shi, Osaka 598-8540 (JP); HIRANO, Keita, Izumisano-shi, Osaka 598-8540 (JP); TAKAYAMA, Hiroyuki, Kakamigahara-shi, Gifu 509-0109 (JP); SATO, Yukihide, Kakamigahara-shi, Gifu 509-0109 (JP)
(74) Representative: Boult Wade Tennant LLP
(86) International application number: PCT/JP2023/013129
(87) International publication number: WO 2023/190844

(57) **Abstract**

Provided is a masking agent for an off-flavor of plant-based protein ingredient, the masking agent containing phenylacetaldehyde and/or 4-hydroxyphenylacetaldehyde.

## Description

### Technical Field

### Related Application

The present application claims the benefit of priority from the Japanese Patent Application No. 2022-058519 filed the Japan Patent Office on March 31, 2022. The basic application for priority claim is incorporated herein by reference in its entirety.

The present invention relates to a masking agent that masks off-flavors of a plant-based protein ingredient and to a production method of the same.

### Background Art

In recent years, demand for plant-based protein ingredients has increased. However, in plant-based protein ingredients, metabolites are produced and accumulated in an extremely large amount as compared with animal protein ingredients, and off-flavors derived therefrom deteriorate the flavor of plant-based protein ingredients. For example, Non-Patent Document 1 indicates that hamburgers prepared using plant-based meat have a strong bitter taste and astringent taste than animal-based hamburgers.

Various methods for reducing the off-flavors of plant-based protein ingredients have been disclosed, such as a method of masking off-flavors by enhancing other flavors (for example, Patent Document 1), a method of masking off-flavors by adding another compound (for example, Patent Documents 2 and 3), and a method of reducing off-flavors by improving the process by which a plant-based protein ingredient is produced (for example, Patent Document 4).

However, masking with other flavors is very likely to impair the flavor of the ingredient itself. In addition, masking by the addition of a compound requires the use of a compound extracted with an organic solvent or a compound designated as a food additive, and use of such masking is contrary to the consumer trend of avoiding the use of additives and raw materials in which an organic solvent is used. Furthermore, the method of improving the production process requires an examination of processes unique to the raw material, and thus lacks versatility. In addition, this method requires the use of additives during the production process, which may not be in line with consumer trends.

Thus, a demand exists for a novel ingredient that can mask the off-flavor of a plant-based protein ingredient. Moreover, the ingredient is preferably prepared from a plant-derived raw material without using an organic solvent.

### Citation List

### Patent Document

Patent Document 1: JP S57-206353 A
Patent Document 2: WO 2019/230903
Patent Document 3: WO 2019/039490
Patent Document 4: WO 2002/028197

### Non-Patent Document

Non-Patent Document 1: KAWAKAMI, Ryoei, et al., "Investigation Research on Plant-Based Meat", Gunma Industrial Technology Center Research Report, 2020

### Summary of Invention

### Technical Problem

Therefore, an object of the present invention is to provide a novel masking agent for masking the off-flavor of a plant-based protein ingredient. A more specific object is to provide a masking agent for masking an off-flavor of a plant-based protein ingredient, the masking agent being prepared from a plant-derived raw material without using an organic solvent.

### Solution to Problem

As a result of intensive studies to solve the above issues, the present inventors surprisingly discovered that phenylacetaldehyde (PAA) and 4-hydroxyphenylacetaldehyde (4HPAA) are produced in trace amounts by enzymatically hydrolyzing a plant-based protein-containing ingredient that contains γ-glutamyl peptides. The present inventors also discovered that PAA and 4HPAA have an effect of masking the off-flavor of plant-based protein ingredients. The present invention was completed based on these findings.

That is, the present invention provides the following aspects:
(1) A masking agent for an off-flavor of a plant-based protein ingredient, the masking agent containing phenylacetaldehyde and/or 4-hydroxyphenylacetaldehyde.
(2) The masking agent according to (1), wherein the plant-based protein ingredient is derived from legumes.
(3) A method for masking an off-flavor of a plant-based protein ingredient, the method including blending phenylacetaldehyde and/or 4-hydroxyphenylacetaldehyde into a food or a food raw material containing the plant-based protein ingredient.
(4) The method according to (3), wherein the plant-based protein ingredient is derived from legumes.
(5) A production method of a masking agent for an off-flavor of a plant-based protein ingredient, the production method including enzymatically treating a plant-based protein-containing ingredient with an enzyme including glutaminase.
(6) The production method according to (5), wherein an enzyme-treated product obtained by enzymatically treating the plant-based protein-containing ingredient with the enzyme including glutaminase contains phenylacetaldehyde and/or 4-hydroxyphenylacetaldehyde.
(7) The production method according to (5) or (6), wherein the plant-based protein-containing ingredient contains a γ-glutamyl peptide.
(8) A production method of a plant-based protein-containing food in which an off-flavor of a plant-based protein ingredient is masked, the production method including enzymatically treating a plant-based protein-containing ingredient with an enzyme including glutaminase, producing an enzyme-treated product containing phenylacetaldehyde and/or 4-hydroxyphenylacetaldehyde, and blending the enzyme-treated product into a food.
(9) The production method according to (8), wherein the plant-based protein-containing food is a beverage containing a plant-based protein derived from legumes.
(10) The production method according to (8) or (9), wherein the plant-based protein-containing ingredient contains a γ-glutamyl peptide.
(11) A production method of phenylacetaldehyde and/or 4-hydroxyphenylacetaldehyde, the production method including a step of treating a γ-glutamyl peptide with an enzyme including glutaminase.

### Advantageous Effects of Invention

According to the present invention, a novel masking agent for masking an off-flavor of a plant-based protein ingredient can be provided. More specifically, the present invention can provide a masking agent for masking an off-flavor of a plant-based protein ingredient, the masking agent being prepared from a natural raw material without using an organic solvent. Even more specifically, the present invention can provide a plant-based protein ingredient whose off-flavor is masked by enzymatically treating the plant-based protein-containing ingredient with glutaminase. In another aspect, the present invention can provide a novel method for producing phenylacetaldehyde and/or 4-hydroxyphenylacetaldehyde.

### Brief Description of Drawings

FIG. 1 is chart presenting the results of Example 2.
FIG. 2 is a diagram indicating a presumed pathway in which phenylacetaldehyde and 4-hydroxyphenylacetaldehyde are produced through glutaminase hydrolysis.
FIG. 3 is a table indicating raw data of a sensory evaluation of Example 4.
FIG. 4 is a table indicating raw data of a sensory evaluation of Example 5.
FIG. 5 is a table indicating raw data of a sensory evaluation of Example 6.
FIG. 6 is a table indicating raw data of a sensory evaluation of Example 7.

### Description of Embodiments

In one aspect, the present invention provides a masking agent for masking an off-flavor of a plant-based protein ingredient, the masking agent containing phenylacetaldehyde (PAA) and/or 4-hydroxyphenylacetaldehyde (4HPAA).

The masking agent of the present aspect contains PAA and/or 4HPAA. The method for procuring or producing PAA and 4HPAA is not particularly limited, and commercially available products may be used, or the PAA and 4HPAA may be produced by a chemical synthesis method or a method using an enzymatic reaction. In one embodiment, the masking agent of the present aspect is produced from a plant-derived material without the use of an organic solvent. In another embodiment, the PAA and 4HPAA are produced from plant-derived raw materials using enzymatic reactions. In specific embodiments, the PAA and 4HPAA are produced by glutaminase treatment of an ingredient derived from legumes, such as, for example, soybeans, lupin beans, alfalfa, white clover, mung beans, adzuki beans, broad beans, peas, chickpeas, kidney beans, lentils, and black-eyed peas. In a more specific embodiment, the PAA and 4HPAA are produced by glutaminase treatment of a soybean-derived ingredient. In an even more specific embodiment, the masking agent of the present aspect is produced according to a below-described method for producing the masking agent.

The content of PAA and/or 4HPAA contained in the masking agent of the present aspect is not particularly limited, and for example may be from 1 ppb to 99%, from 10 ppb to 75%, from 100 ppb to 50%, from 1 ppm to 30%, from 100 ppm to 10%, or from 1 to 5%. Moreover, 100% pure products of PAA and/or 4HPAA may be used as the masking agent of the present aspect. In a more specific embodiment, the masking agent of the present aspect may be produced from a plant-derived raw material by an enzymatic reaction and then used as is, and in this case, the content of the PAA and/or 4HPAA may be, for example, from 10 ppb to 1 ppm.

In one embodiment, examples of the origin of the plant-based protein ingredient to be treated by the masking agent of the present aspect include legumes, such as soybeans, lupin beans, alfalfa, white clover, mung beans, adzuki beans, broad beans, peas, chickpeas, kidney beans, lentils, and black-eyed peas; seeds, such as sesame seeds, canola seeds, coconut seeds, and almond seeds; grains, such as corn, buckwheat, wheat, and rice; vegetables; and fruits. In a more specific embodiment, the plant-based protein ingredient to be treated by the masking agent of the present aspect is derived from legumes. In yet a more specific embodiment, the plant-based protein ingredient to be treated by the masking agent of the present aspect is derived from soybeans, lupin beans, alfalfa, white clover, mung beans, adzuki beans, broad beans, peas, chickpeas, kidney beans, lentils, black-eyed peas, or combinations thereof. In yet an even more specific embodiment, the plant-based protein ingredient to be treated by the masking agent of the present aspect is derived from soybeans.

For example, in the case of a plant-based protein ingredient derived from legumes, examples include a protein and/or a decomposition product thereof, and the method for producing the protein and/or decomposition product thereof is not particularly limited as long as the protein and/or decomposition product is obtained by separating and purifying the protein and/or decomposition product contained in the legumes. In addition, the legume-derived protein and/or the decomposition product thereof may be a commercially available product. For example, in the case of soy proteins, examples include defatted soybeans obtained by defatting whole soybeans or the like using an organic solvent such as hexane or ethanol, soy milk obtained by extracting proteins from whole soybeans or defatted soybeans using water, isolated soy proteins obtained by a method such as acid precipitation or alcohol precipitation from soy milk, concentrated soy proteins, soybean whey, and concentrated soybean whey. A mixture thereof may also be used. Lupin beans, alfalfa, white clover, mung beans, adzuki beans, broad beans, peas, chickpeas, kidney beans, lentils, black-eyed peas, and the like may also be separated and purified in the same manner as soybeans. Concentrated products thereof, whey products thereof, or concentrated whey products thereof may also be used. A mixture of these may also be used.

The content of the plant-based protein in the plant-based protein ingredient of the present aspect is not particularly limited. In one embodiment, the content of the plant-based protein in the plant-based protein ingredient of the present aspect is from 10 to 100%, and for example, may be 10% or more, 20% or more, 30% or more, 40% or more, 50% or more, 60% or more, 70% or more, 80% or more, 90% or more, 95% or more, or 99% or more. In one embodiment, the plant-based protein ingredient of the present aspect may contain carbohydrates, lipids, minerals, and the like in addition to protein. In a specific embodiment, the lipid content in the plant-based protein ingredient is, for example, 5% or less, 2% or less, 1% or less, or 0.5% or less.

As used herein, the term "off-flavor" refers to an unpleasant odor and an unpleasant taste derived from a plant-based protein. Specific examples of the off-flavor include a "protein odor", an "astringent taste", a "harsh taste", and "bitterness" derived from plant-based proteins such as soy protein and wheat protein; a "bean odor" and a "grassy odor" derived from legume proteins such as soy proteins and pea proteins; and a "grain odor" derived from grain proteins such as wheat proteins. In a specific embodiment, the masking agent of the present aspect has the effect of masking the "bean odor" and "grassy odor" derived from legume proteins. In a more specific embodiment, the masking agent of the present aspect has a masking effect on the "soybean odor" and "grassy odor" of soy protein.

Other raw materials may be added to the masking agent of the present aspect as long as the function of the masking agent is not impaired, but other such raw materials are not required to be added. Examples of such other raw materials include seasonings, acidulants, sweeteners, spices, colorants, flavors, salts, sugars, antioxidants, vitamins, stabilizers, thickeners, carriers, excipients, lubricants, surfactants, propellants, preservatives, chelating agents, and pH adjusters. In one embodiment, the masking agent of the present aspect has a salt concentration as sodium chloride of from 0 to 10%, and for example, the salt concentration may be 9.5% or less, 7.5% or less, 4.5% or less, 3% or less, 2% or less, or 1% or less.

The form of the masking agent of the present aspect is not particularly limited, and examples thereof include solids such as powders, granules, and pellets; semi-solids such as pastes; and liquids such as solutions, suspensions, and emulsions. In a specific embodiment, as the masking agent, for example, a masking agent produced from a plant-derived raw material through an enzymatic reaction may be used as is, or a masking agent that is crudely purified liquid or a powder obtained by drying the liquid thereof may be used.

In one aspect, the present invention provides a method for masking an off-flavor of a plant-based protein ingredient, the masking method including blending PAA and/or 4HPAA into a food or a food raw material containing the plant-based protein ingredient. In one embodiment, PAA and/or 4HPAA may be blended in the form of a masking agent of the aspect described above. The term "food" as used herein includes foods and beverages unless otherwise specified. Note that the matters described in the above-described aspect of the masking agent also apply to the present aspect.

The food or food raw material of the present aspect is not particularly limited as long as the food or food raw material contains a plant-based protein ingredient. The food or food raw material containing a plant-based protein ingredient also includes a case in which the plant-based protein ingredient itself is a food or food raw material. Examples of foods in the present aspect include beverages such as soy milk beverages, milk substitute beverages, plant-based protein-containing beverages, and nutritional drinks; processed meat foods such as ham, sausage, and hamburger; processed meat substitutes that use soy protein, pea protein, and the like; processed seafoods such as steamed seasoned fish paste and tube-shaped fish paste cake; processed seafood substitutes in which soy protein, pea protein, or the like is used; dairy products such as butter, fermented milk, powdered milk, cream, cheese, yogurt, and ice cream; dairy alternatives such as soybean cream, soybean cheese, soybean yogurt and soybean ice cream; confectioneries or confectionery materials such as snacks, cookies, whipped creams, custard cream, flour paste, cakes, puddings, jellies, sweet bun (manju), and dumplings (dango); seasonings such as sauces and mayonnaise; and breads and noodles. In one embodiment, the food of the present aspect contains a protein derived from legumes. In a specific embodiment, the food of the present aspect contains a protein derived from soybeans, lupin beans, alfalfa, white clover, mung beans, adzuki beans, broad beans, peas, chickpeas, kidney beans, lentils, black-eyed peas, or combinations thereof. In a more specific embodiment, the food of the present aspect contains a protein derived from soybeans. In another embodiment, the food of the present aspect is a legume-derived protein ingredient itself, and examples thereof include soy milk, low-fat soy milk, powdered soy milk, dairy-substitute milk, isolated soy protein, and isolated pea protein.

In one embodiment, the food of the present aspect may be a food composition. In other embodiment, the food of the present aspect may be a quasi-drug instead of a food. The food composition or quasi-drug may also contain a carrier or an additive. Examples thereof include, but are not limited to, a surfactant, an excipient, a colorant, a flavoring agent, a preservative, a stabilizer, a buffer, a suspending agent, an isotonic agent, a binder, a disintegrating agent, a lubricant, a fluidity promoter, and a taste masking agent, and furthermore, a commonly used carrier can be appropriately used. The form of the food composition or quasi-drug is not particularly limited, and examples thereof include a concentrated liquid, a powder, a granule, a tablet, a pill, a gum, a candy, a capsule, a paste, a jelly, and a drink.

In addition to adding PAA and/or 4HPAA to the food or food raw material, the term "blending" referred to herein also includes, for example, subjecting the plant-based protein ingredient contained in a food or food raw material to a glutaminase treatment to produce PAA and/or 4HPAA in a food or food raw material.

In the method of the present aspect, the amount of PAA and/or 4HPAA to be blended in the food or food raw material is not particularly limited, and can be appropriately adjusted according to the type and strength of the off-flavor to be masked. As an example of the blending amount, the PAA and/or 4HPAA may be blended such that the food or the food raw material contains the PAA and/or 4HPAA at an amount of from 0.1 ppb to 10 ppm, or from 1 ppb to 10 ppm, or for example, from 5 ppb to 5 ppm, from 10 ppb to 1 ppm, or from 50 ppb to 0.5 ppm.

The timing and the number of times at which the PAA and/or 4HPAA is blended in a food or food raw material are not particularly limited. In addition, the PAA and/or 4HPAA may be blended with other food raw materials or may be blended alone. For example, the PAA and/or 4HPAA may be blended at the beginning, in the middle, or at the end. Moreover, for example, the PAA and/or 4HPAA may be blended once, or the blending thereof may be divided into two or more times. In addition, a step of subjecting the plant-based protein ingredient contained in the food or food raw material to a glutaminase treatment to produce PAA and/or 4HPAA in the food or food raw material may be combined with a step of adding PAA and/or 4HPAA to the food or food raw material.

In one aspect, the present invention provides a method for producing a masking agent for masking an off-flavor of a plant-based protein ingredient, the production method including enzymatically treating a plant-based protein-containing ingredient with an enzyme including glutaminase. Note that the matters described in each of the above aspects also apply to the present aspect.

In one embodiment, examples of the origin of the plant-based protein-containing ingredient to be enzymatically treated by an enzyme including glutaminase in the production method of the present aspect include legumes, such as soybeans, lupin beans, alfalfa, white clover, mung beans, adzuki beans, broad beans, peas, chickpeas, kidney beans, lentils, and black-eyed peas; seeds, such as sesame seeds, canola seeds, coconut seeds, and almond seeds; grains, such as corn, buckwheat, wheat, and rice; vegetables; and fruits. In a more specific embodiment, the plant-based protein-containing ingredient of the present aspect is derived from legumes. In yet a more specific embodiment, the plant-based protein-containing ingredient of the present aspect is derived from soybeans, lupin beans, alfalfa, white clover, mung beans, adzuki beans, broad beans, peas, chickpeas, kidney beans, lentils, black-eyed peas, or combinations thereof. In yet an even more specific embodiment, the plant-based protein-containing ingredient is derived from soybeans.

For example, in the case of a plant-based protein ingredient derived from legumes, examples include a protein and/or a decomposition product thereof, and the method for producing the protein and/or decomposition product thereof is not particularly limited as long as the protein and/or decomposition product is obtained by separating and purifying the protein and/or decomposition product contained in the legumes. In addition, the legume-derived protein and/or the decomposition product thereof may be a commercially available product. For example, in the case of soy proteins, examples include defatted soybeans obtained by defatting whole soybeans or the like using an organic solvent such as hexane or ethanol, soy milk obtained by extracting proteins from whole soybeans or defatted soybeans using water, isolated soy proteins obtained by a method such as acid precipitation or alcohol precipitation from soy milk, concentrated soy proteins, soybean whey, and concentrated soybean whey. A mixture thereof may also be used. Lupin beans, alfalfa, white clover, mung beans, adzuki beans, broad beans, peas, chickpeas, kidney beans, lentils, black-eyed peas, and the like may also be separated and purified in the same manner as soybeans. Concentrated products thereof, whey products thereof, or concentrated whey products thereof may also be used. A mixture of these may also be used.

The content of the plant-based protein in the plant-based protein-containing ingredient of the present aspect is not particularly limited. In one embodiment, the content of the plant-based protein in the plant-based protein-containing ingredient of the present aspect is from 10 to 100%, and for example, may be 10% or more, 20% or more, 30% or more, 40% or more, 50% or more, 60% or more, 70% or more, 80% or more, 90% or more, 95% or more, or 99% or more. In one embodiment, the plant-based protein-containing ingredient of the present aspect may contain carbohydrates, lipids, minerals, and the like in addition to protein. In a specific embodiment, the lipid content in the plant-based protein-containing ingredient is, for example, 5% or less, 2% or less, 1% or less, or 0.5% or less.

The term "glutaminase" as used herein refers to an enzyme that catalyzes a reaction for producing glutamic acid by cleaving the amide bond at the γ-position of glutamine. Any enzyme, microorganism, or the like having this enzyme activity can be used in the present aspect as long as the enzyme, microorganism, or the like has this enzyme activity. For example, a commercially available glutaminase preparation may be used. Examples of commercially available glutaminase preparations include glutaminase SD-C100S, glutaminase SD-100NA, and glutaminase F "Amano" 100 (all of which are available from Amano Enzyme Inc.). In a specific embodiment, the glutaminase used in the present aspect has γ-glutamyl transferase activity. In another embodiment, the glutaminase used in the present aspect is derived from bacteria. In a specific embodiment, the glutaminase used in the present aspect is derived from bacteria of the genus Bacillus, and for example, may be derived from Bacillus amyloliquefaciens. In another embodiment, the glutaminase used in the present aspect is not derived from a filamentous fungus.

In one embodiment, in the production method of the present aspect, an enzyme other than glutaminase may or may not be used in combination with the glutaminase. Examples of the enzyme that may be used in combination include amylase and protease. For example, an enzyme preparation containing glutaminase and amylase, or glutaminase and protease may be used in the production method of the present aspect. As one example, when decomposing of saccharides and proteins contained in the plant-based protein-containing ingredient is desired, amylase or protease is preferably used in combination. For example, the enzyme to be used in combination may be an enzyme derived from a glutaminase-producing bacterium or may be an enzyme derived from another source. As another example, when decomposition of saccharides or proteins more than necessary is not desired, it is preferable not to use amylase or protease in combination.

The reaction pH and the reaction temperature of the enzyme treatment of the present aspect need only be set in accordance with the characteristics of the enzyme to be used, and the reaction need only be usually carried out at a reaction pH near the optimum pH and at a reaction temperature near the optimum temperature. The reaction can be generally carried out at a pH in a range of from 2 to 10. The reaction can be generally carried out at a reaction temperature of from 20 to 80°C and preferably from 40 to 60°C. The reaction may be carried out continuously in a bioreactor or the like. After the reaction, the reaction product may be heated at a temperature sufficient for deactivating the enzyme (about 60 to 170°C) to thereby deactivate the remaining enzyme activity.

The usage amount of the enzyme in the present aspect may be set according to details such as the properties of the enzyme to be used. Specific examples of the usage amount of the enzyme per 1 g of the plant-based protein-containing ingredient include from 0.0001 U to 100 U, and for example, from 0.001 U to 10 U, or from 0.01 to 1 U. One unit (U) of the enzyme activity (U) referred to herein is defined as the amount of enzyme that produces 1 µmοl of L-glutamic acid per minute when a 1% (w/v) L-glutamine solution (0.1 mol/L acetate buffer (pH of 6.0)) is used as a substrate and the reaction is carried out at 37°C for 10 minutes.

In one embodiment, the salt concentration as sodium chloride during the reaction in the present aspect is from 0 to 10%, and for example, the salt concentration may be 9.5% or less, 7.5% or less, 4.5% or less, 3% or less, 2% or less, or 1% or less.

The timing and frequency of the enzymatic reaction in the method of the present aspect are not particularly limited. The enzymatic reaction may be carried out once or may be divided into two or more times and carried out. In addition, the glutaminase that is used when the enzymatic reaction is carried out a plurality of times may be the same or different each time.

In one embodiment, the masking agent obtained by the production method of the present aspect contains PAA and/or 4HPAA.

In one embodiment, the plant-based protein-containing ingredient to be enzymatically treated with the enzyme including glutaminase in the production method of the present aspect contains a γ-glutamyl peptide. In a specific embodiment, the plant-based protein-containing ingredient of the present aspect contains a γ-glutamyl peptide represented by γ-Glu-Phe-Xₙ and/or γ-Glu-Tyr-Xₙ. As used herein, unless otherwise indicated, "Glu" means glutamic acid, "Phe" means phenylalanine, "Tyr" means tyrosine, "X" means any amino acid or amino acid derivative, and "n" means an integer greater than or equal to 0. In the present specification, for example, "γ-Glu-Phe" means that Glu and Phe are bonded via a carboxyl group at the γ-position of glutamic acid. In one embodiment, "n" is from 0 to 20, and may be, for example, from 0 to 10, or from 0 to 5. In a more specific embodiment, the plant-based protein-containing ingredient of the present aspect contains a γ-glutamyl peptide represented by γ-Glu-Phe and/or γ-Glu-Tyr. Specific examples thereof include the plants that are said to contain a γ-glutamyl peptide as indicated by KASAI, Takanori et al. (1975) in the Journal of Agricultural Chemical Society of Japan, Vol. 49, No. 6, pp. 313-316.

In one embodiment, the plant-based protein-containing ingredient to be enzymatically treated and the plant-based protein ingredient to be masked are the same ingredient. In another embodiment, the plant-based protein-containing ingredient to be enzymatically treated and the plant-based protein ingredient to be masked are different ingredients.

In one aspect, the present invention provides a method for producing a plant-based protein-containing food in which an off-flavor of a plant-based protein ingredient is masked, the production method including enzymatically treating a plant-based protein-containing ingredient with an enzyme including glutaminase to produce an enzyme-treated product containing PAA and/or 4HPAA, and blending the enzyme-treated product into a food. Note that the matters described in each of the above aspects also apply to the present aspect.

In addition to adding an enzyme-treated product containing PAA and/or 4HPAA to a food as a food raw material, the term "blending" in the present aspect also includes, for example, subjecting the plant-based protein-containing ingredient contained in the food to an enzymatic treatment with an enzyme including glutaminase to produce an enzyme-treated product containing PAA and/or 4HPAA in the food. In one embodiment, the production method of the present aspect includes adding, as a food raw material, an enzyme-treated product containing PAA and/or 4HPAA to a food. In another embodiment, the production method of the present aspect includes enzymatically treating a plant-based protein-containing ingredient contained in a food with an enzyme including glutaminase to produce an enzyme-treated product containing PAA and/or 4HPAA in the food.

The timing and the number times at which the enzyme-treated product containing PAA and/or 4HPAA is blended as a food raw material into a food are not particularly limited. In addition, the enzyme-treated product containing PAA and/or 4HPAA may be blended with other food raw materials or may be blended alone. For example, the enzyme-treated product may be blended at the beginning, in the middle, or at the end. In addition, for example, the enzyme-treated product may be blended once, or the blending thereof may be divided into two or more times. Moreover, a step of adding, as a food raw material, an enzyme-treated product containing PAA and/or 4HPAA to a food may be combined with a step of subjecting the plant-based protein-containing ingredient contained in the food to an enzymatic treatment with an enzyme including glutaminase to produce an enzyme-treated product containing PAA and/or 4HPAA in the food.

In another aspect, the present invention provides a method for producing phenylacetaldehyde and/or 4-hydroxyphenylacetaldehyde, the production method including a step of treating a γ-glutamyl peptide with an enzyme including glutaminase.

In a specific embodiment, the γ-glutamyl peptide treated by the method of the present aspect includes γ-glutamyl peptides represented by γ-Glu-Phe-Xₙ and/or γ-Glu-Tyr-Xₙ. In one embodiment, "n" is from 0 to 20, and for example, is from 0 to 10, or from 0 to 5. In a more specific embodiment, the γ-glutamyl peptide of the present aspect includes γ-glutamyl peptides represented by γ-Glu-Phe and/or γ-Glu-Tyr.

Any enzyme, microorganism, or the like having glutaminase activity can be used in the present aspect as long as the enzyme, microorganism, or the like has this enzyme activity. For example, a commercially available glutaminase preparation may be used. Examples of commercially available glutaminase preparations include glutaminase SD-C100S, glutaminase SD-100NA, and glutaminase F "Amano" 100 (all of which are available from Amano Enzyme Inc.). In a specific embodiment, the glutaminase used in the present aspect has γ-glutamyl transferase activity. In another embodiment, the glutaminase used in the present aspect is derived from bacteria. In a specific embodiment, the glutaminase used in the present aspect is derived from bacteria of the genus Bacillus, and for example, may be derived from Bacillus amyloliquefaciens. In another embodiment, the glutaminase used in the present aspect is not derived from a filamentous fungus.

In one embodiment, in the production method of the present aspect, an enzyme other than glutaminase may or may not be used in combination with the glutaminase. Examples of the enzyme that may be used in combination include amylase and protease. For example, an enzyme preparation containing glutaminase and amylase, or glutaminase and protease may be used in the production method of the present aspect. As one example, when decomposing of saccharides and proteins contained in the plant-based protein-containing ingredient is desired, amylase or protease is preferably used in combination. For example, the enzyme to be used in combination may be an enzyme derived from a glutaminase-producing bacterium or may be an enzyme derived from another source. As another example, when decomposition of saccharides or proteins more than necessary is not desired, it is preferable not to use amylase or protease in combination.

The reaction pH and the reaction temperature of the enzyme treatment of the present aspect need only be set in accordance with the characteristics of the enzyme to be used, and the reaction need only be usually carried out at a reaction pH near the optimum pH and at a reaction temperature near the optimum temperature. The reaction can be generally carried out at a pH in a range of from 2 to 10. The reaction can be generally carried out at a reaction temperature of from 20 to 80°C and preferably from 40 to 60°C. The reaction may be carried out continuously in a bioreactor or the like. After the reaction, the reaction product may be heated at a temperature sufficient for deactivating the enzyme (about 60 to 170°C) to thereby deactivate the remaining enzyme activity.

In one embodiment, the salt concentration as sodium chloride during the reaction in the present aspect is from 0 to 10%, and for example, the salt concentration may be 9.5% or less, 7.5% or less, 4.5% or less, 3% or less, 2% or less, or 1% or less.

The timing and frequency of the enzymatic reaction in the method of the present aspect are not particularly limited. The enzymatic reaction may be carried out once or may be divided into two or more times and carried out. Moreover, when the enzymatic reaction is carried out multiple times, the glutaminase that is used each time may be the same or different.

The phenylacetaldehyde and/or the 4-hydroxyphenylacetaldehyde obtained in the present aspect can be used as a raw material for a food, a flavoring, or the like.

### Examples

Hereinafter, embodiments of the present invention will be more specifically described by examples and the like. Note that unless otherwise specified, units such as "%" in the examples are on a mass basis.

### Example 1: Glutaminase Treatment of Soy Milk

Glutaminase (product name: Glutaminase SD-C100S, available from Amano Enzyme Inc.) was added to low-fat soy milk (available from Fuji Oil Co., Ltd.) at an amount of 0.1% based on the weight of the soy milk, and the mixture was allowed to react at 50°C for 1 hour and was then boiled for 15 minutes to deactivate the enzyme.

The obtained enzyme-treated low-fat soy milk and the low-fat soy milk before treatment were evaluated for flavor by ten trained panelists. The panelists confirmed that the flavor of soy milk was improved by the glutaminase treatment.

### Example 2: GC/MS Analysis of Phenylacetaldehyde

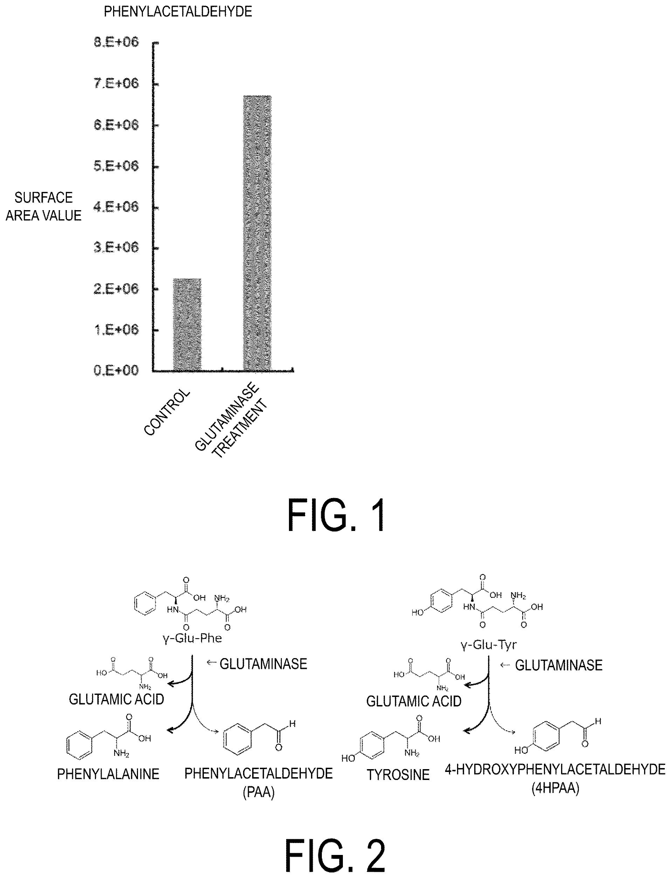

A gas chromatography-mass spectrometry (GC/MS) analysis was carried out to confirm whether a change occurred in the aroma components in the soy milk between the soy milk without treatment and the soy milk after the glutaminase treatment. The results are presented in FIG. 1. The results confirmed that phenylacetaldehyde (PAA) increased in the glutaminase-treated soy milk. In addition, the results confirmed that 4-phenylacetaldehyde (4HPAA) also increased in the glutaminase-treated soy milk.

### Example 3: Analysis of Glutaminase Hydrolysate-Derived Aldehydes

Through an analysis of the peptide content in the soy milk, it was confirmed that γ-glutamyl peptides in the soy milk were degraded after the glutaminase treatment. Therefore, as presented in FIG. 2, it was anticipated that PAA would be produced as a by-product of the hydrolysis reaction of γ-Glu-Phe and that 4HPAA would be produced as a by-product of the hydrolysis reaction of γ-Glu-Tyr. To confirm this, standard samples of γ-Glu-Phe and γ-Glu-Tyr were enzymatically treated with 0.1% glutaminase at an initial peptide concentration of 1000 ppm at 50°C for 1 hour, and then the concentrations of PAA and 4HPAA were measured. The results are presented in Table 1.

**[Table 1]**

| Standard Sample | Enzyme Treatment | Initial Peptide Concentration (ppm) | Produced Aldehyde | Aldehyde Concentration (ppb) in Solution | Produced Aldehyde (ppb) | Aldehyde Conversion Rate (%) |
|---|---|---|---|---|---|---|
| γ-Glu-Phe | - | 1000 | PAA | 11.2 | 42.4 | **0.00424** |
| | + | | | 53.6 | | |
| γ-Glu-Tyr | - | 1000 | 4HPAA | ND* | 203 | **0.0203** |
| | + | | | 203 | | |

| | | | | | | |
|---|---|---|---|---|---|---|
| *ND: Not detected | | | | | | |

It was confirmed that trace amounts of the aldehydes were produced by hydrolyzing the γ-glutamyl peptides with glutaminase.

### Example 4: Addition of PAA and 4HPAA to Processed Soy Milk or Soy Protein Solution

A 5% solution was prepared using a processed soy milk powder (Soyafit 2000, available from Fuji Oil Co., Ltd.) or an isolated soy protein (Fujipro E, available from Fuji Oil Co., Ltd.). PAA and 4HPAA were each dissolved in propylene glycol to a concentration of 0.1%. The 5% solution of processed soy milk or isolated soy protein, the PAA solution, and the 4HPAA solution were mixed such that the PAA concentration was 6 ppb and the 4HPAA concentration was 30 ppb, and a sample A was prepared from the processed soy milk powder, and a sample B was prepared from the isolated soy protein.

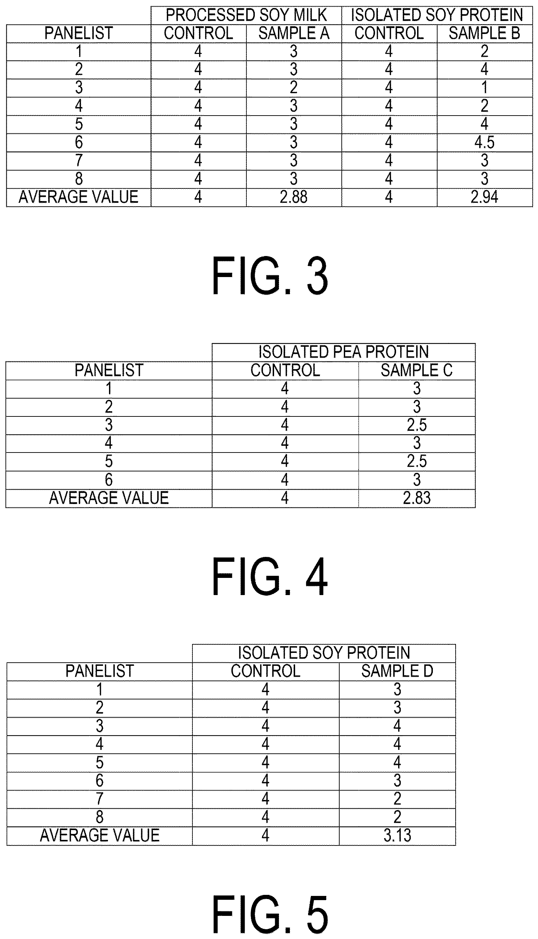

Sensory evaluations were carried out by eight trained panelists. A 5% solution of processed soy milk or isolated soy protein to which an aldehyde was not added was used as a control. The soybean odor sensed from the control was given a score of 4 points, and the soybean odor sensed from the samples A and B was evaluated with scores ranging from 1 (weak) to 7 (strong). If the average score was lower than that of the control, the sample was determined to be passing. The results are presented in FIG. 3 and Table 2.

**[Table 2]**

| | Average Value (points) |
|---|---|
| Control | 4 |
| Sample A | 2.88 |
| Sample B | 2.94 |

As presented in Table 2, Sample A and Sample B to which PAA and 4HPAA were added exhibited weaker soybean odor than the control. Note that while the effect of suppressing soybean odor by adding only PAA or only 4HPAA was weaker than the effect obtained by adding both PAA and 4HPAA, adding only PAA or only 4HPAA was confirmed to have an effect of suppressing soybean odor.

### Example 5: Addition of PAA and 4HPAA to Isolated Pea Protein Solution

A 5% solution was prepared with isolated pea protein (Empro E86, available from Emsland GmbH). PAA and 4HPAA were each dissolved in propylene glycol to a concentration of 0.1%. The 5% solution of isolated pea protein, the PAA solution, and the 4HPAA solution were mixed to prepare a Sample C in which the concentration of PAA was 6 ppb and the concentration of 4HPAA was 30 ppb.

Sensory evaluations were carried out by six trained panelists. A 5% solution of the isolated pea protein to which no aldehyde was added was used as a control. The pea odor sensed from the control was given a score of 4 points, and the pea odor sensed from the Sample C, which was a solution to which an aldehyde was added, was evaluated with scores ranging from 1 (weak) to 7 (strong). If the average score was lower than that of the control, the sample was determined to be passing. The results are presented in Table 3 and FIG. 4.

**Table 3]**

| | Average Value (points) |
|---|---|
| Control | 4 |
| Sample C | 2.83 |

As presented in Table 3, the Sample C to which PAA and 4HPAA were added had a weaker pea odor than the control.

### Example 6: Addition of Concentrated Soybean Whey Enzyme-Treated Product to Isolated Soy Protein Solution

A concentrated soybean whey was adjusted to a pH of 7, and glutaminase was added thereto at a concentration of 0.1% in relation to the concentrated soybean whey. The mixture was then subjected to an enzyme treatment at a reaction temperature of 50°C for 1 hour. The obtained concentrated soybean whey enzyme-treated product was added to a 10% solution of isolated soy protein (Fujipro E, available from Fuji Oil Co., Ltd.) such that the concentration of the concentrated soybean whey enzyme-treated product therein was 5%, and a Sample D was thereby prepared.

A sensory evaluation was carried out in the same manner as in Example 4 by eight trained panelists using, as a control, a 10% isolated soy protein solution to which concentrated soybean whey not subjected to the enzyme treatment was added at a concentration of 5%. The results are presented in FIG. 5 and Table 4.

**[Table 4]**

| | Average Value (points) |
|---|---|
| Control | 4 |
| Sample D | 3.13 |

As presented in Table 4, the Sample D to which the concentrated soybean whey enzyme-treated product was added exhibited a weaker soybean odor than the control.

### Example 7: Addition of Concentrated Soybean Whey Enzyme-Treated Product to Isolated Pea Protein Solution

A concentrated soybean whey enzyme-treated product obtained in the same manner as in Example 6 was added to a 5% solution of isolated pea protein (Empro E86, available from Emsland GmbH) such that the concentration of the concentrated soybean whey enzyme-treated product therein was 5%, and a Sample E was thereby prepared.

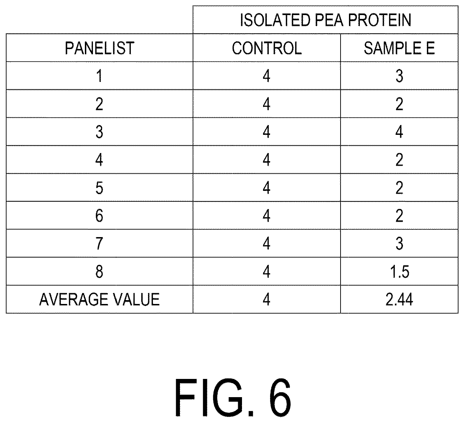

A sensory evaluation was carried out in the same manner as in Example 5 by eight trained panelists using, as a control, a 5% isolated pea protein solution to which concentrated soybean whey not subjected to the enzyme treatment was added at a concentration of 5%. The results are presented in FIG. 6 and Table 5.

**[Table 5]**

| | Average Value (points) |
|---|---|
| Control | 4 |
| Sample E | 2.44 |

As presented in Table 5, Sample E to which the concentrated soybean whey enzyme-treated product was added exhibited a weaker pea odor than the control.

### Industrial Applicability

According to the present invention, a masking agent that masks the off-flavor of a plant-based protein ingredient can be provided. The masking agent can be used in foods, food compositions, quasi-drugs, and the like.

## Claims

1. A masking agent for an off-flavor of a plant-based protein ingredient, the masking agent comprising phenylacetaldehyde and/or 4-hydroxyphenylacetaldehyde.

2. The masking agent according to claim 1, wherein the plant-based protein ingredient is derived from legumes.

3. A method for masking an off-flavor of a plant-based protein ingredient, the method comprising blending phenylacetaldehyde and/or 4-hydroxyphenylacetaldehyde into a food or a food raw material containing the plant-based protein ingredient.

4. The method according to claim 3, wherein the plant-based protein ingredient is derived from legumes.

5. A production method of a masking agent for an off-flavor of a plant-based protein ingredient, the production method comprising enzymatically treating a plant-based protein-containing ingredient with an enzyme including glutaminase.

6. The production method according to claim 5, wherein an enzyme-treated product obtained by enzymatically treating the plant-based protein-containing ingredient with the enzyme including glutaminase contains phenylacetaldehyde and/or 4-hydroxyphenylacetaldehyde.

7. The production method according to claim 5 or 6, wherein the plant-based protein-containing ingredient comprises a γ-glutamyl peptide.

8. A production method of a plant-based protein-containing food in which an off-flavor of a plant-based protein ingredient is masked, the production method comprising:
enzymatically treating a plant-based protein-containing ingredient with an enzyme including glutaminase;
producing an enzyme-treated product containing phenylacetaldehyde and/or 4-hydroxyphenylacetaldehyde; and
blending the enzyme-treated product into a food.

9. The production method according to claim 8, wherein the plant-based protein-containing food is a beverage containing a plant-based protein derived from legumes.

10. The production method of a plant-based protein-containing food according to claim 8 or 9, wherein the plant-based protein-containing ingredient comprises a γ-glutamyl peptide.

11. A production method of phenylacetaldehyde and/or 4-hydroxyphenylacetaldehyde, the production method comprising a step of treating a γ-glutamyl peptide with an enzyme including glutaminase.
